# EUROPEAN PATENT APPLICATION

(11) **EP 3 862 745 A1**
(43) Date of publication of application: **11.08.2021**
(21) Application number: 19868450.8
(22) Date of filing: 27.09.2019
(51) Int. Cl.: G01N 21/27, G01N 21/05, G01N 35/08, G01N 37/00

(54) **TRACE SUBSTANCE OPTICAL MEASURING INSTRUMENT AND MEASURING METHOD**

(30) Priority: 05.10.2018 JP 2018189758
(71) Applicant: Provigate Inc., Tokyo 113-0033 (JP)
(72) Inventor: KATAYAMA, Norikazu, Tokyo 113-0033 (JP); ITO, Narushi, Tokyo 113-0033 (JP)
(74) Representative: Michalski Hüttermann & Partner Patentanwälte mbB
(86) International application number: PCT/JP2019/038151
(87) International publication number: WO 2020/071273

(57) **Abstract**

Provided is a device for measuring a substance in a solution, provided with: a light source; a micro flow passage including at least a portion of an optical path of light emitted from the light source, and extending along said optical path; and a light receiver for detecting light that has passed through the micro flow passage.

## Description

### Technical Field

The present disclosure relates to optical measurement instruments and measurement methods for measuring trace substances.

### Background Art

The concentration and other properties of a substance in solution can be estimated or identified by optical measurements.

So far, in order to measure the concentration of a measurement target substance in a solution, the absorbance measurement method is used as an example. As measurement apparatus, cells and cuvettes for spectrophotometers are commonly used, which require a relatively large volume of a few mL of solution using a transparent cell such as a square glass or quartz or plastic having an optical path length of about 10 mm. Recently, there has been a desire to measure trace substances from trace samples such as interstitial fluid and tear fluid with high sensitivity.

### Summary of Invention

### Technical Problem

When the concentration of the target substance to be measured is low, it is impossible to increase the amount of liquid by dilution, and it is difficult to obtain a certain optical path length and measure the concentration of the substance from a sample of a trace liquid amount with high sensitivity.

### Solution to Problem

According to one embodiment of the present disclosure, an apparatus for measuring a substance in a solution comprises a light source, a micro flow channel including at least a portion of an optical path of light emitted from the light source and extending along the optical path, and a photodetector for detecting light that has passed through the micro flow channel.

### Description of Drawings

[FIG. 1] Cross-sectional view of an optical measurement instrument according to an embodiment of the present disclosure.
[FIG. 2] Cross-sectional view of an optical measurement instrument according to an embodiment of the present disclosure.
[FIG. 3] Cross-sectional view of an optical measurement instrument according to an embodiment of the present disclosure.
[FIG. 4] Cross-sectional view of an optical measurement instrument according to an embodiment of the present disclosure.
[FIG. 5] Cross-sectional view of an optical measurement instrument according to an embodiment of the present disclosure.
[FIG. 6] Block diagram showing a configuration of an optical measurement instrument according to an embodiment of the present disclosure.
[FIG. 7] Block diagram showing a configuration of an optical measurement instrument according to an embodiment of the present disclosure.
[FIG. 8] Block diagram showing a configuration of an optical measurement instrument according to an embodiment of the present disclosure.
[FIG. 9] Perspective view of an optical measurement instrument according to an embodiment of the present disclosure.
[FIG. 10] Graph illustrating an embodiment of the present disclosure.
[FIG. 11] Perspective view of an optical measurement instrument according to an embodiment of the present disclosure.
[FIG. 12] Flowchart of an optical measurement according to an embodiment of the present disclosure.
[FIG. 13] Flowchart of an optical measurement according to an embodiment of the present disclosure.

### Description of Embodiments

### 1. Measurement apparatus

In some embodiments of the present disclosure, there is provided an apparatus for measuring properties such as physical properties and the concentration of a substance in a solution by an optical method. The measurement apparatus has a light source, a photodetector and a flow channel. All or a portion of the light emitted from the light source enters the flow channel. All or a portion of the light has passed through the flow channel enters the photodetector. The flow channel may be formed to be elongated in the longitudinal direction. The optical path may be defined along the longitudinal direction of the flow channel. The light may propagate within the flow channel along the longitudinal direction of the flow channel. The light that has passed through the channel is received by the photodetector. The light source may be disposed outside of the flow channel, may be disposed on a wall of the flow channel, or may be disposed within the flow channel. The photodetector may be disposed outside of the flow channel, may be disposed on a wall of the flow channel, or may be disposed within the flow channel.

The optical method used may be a spectroscopic method, an absorbance spectroscopy, or other methods. The measurement apparatus may have a configuration of a colorimeter or an absorbance spectroscope for measuring the absorbance (or transmittance) of light of a specific wavelength, and may have a configuration of a colorimeter, a color difference meter, or the like. The measurement apparatus may or may not use practically specific scientific methods. The measurement apparatus may be configured to determine a characteristic such as an unknown concentration from the output of the photodetector at the time of measurement based on the relationship between the output of the photodetector and the characteristic such as the concentration.

The light source is configured to emit electromagnetic waves. In some embodiments, the wavelength of the light source may be determined according to the absorption properties of the substance as a measurement target. In some embodiments, the wavelength of the light source may be determined based on a factor other than the absorbance characteristic of the substance as measurement target, and may be determined based on a plurality of factors.

The wavelength of the light source may be visible in some embodiments, infrared in some embodiments, or ultraviolet in some embodiments. The wavelength of the light source may be between 200 and 1500 nm (nanometers, hereinafter the same) and may be between 560 and 700 nm.

In some embodiments, the light source may be a light emitting diode (LED). In some embodiments, the light source may be a laser light source. In some embodiments, the light emission of the light source may be electroluminescence, organic EL (electroluminescence, hereinafter the same), inorganic EL, chemiluminescence, electrochemiluminescence.

In some embodiments, the flow channel may be a micro flow channel. In some embodiments, the volume of the flow channel may be smaller than or equal to a value such as 100 *µ*L (microliter, hereinafter the same), 50 *µ*L, 20 *µ*L, 10 *µ*L, 5 *µ*L and the like.

The dimension of the cross-section of the flow channel, e.g., the cross-section perpendicular to the optical path of the portion in which the optical path is located, may be smaller than or equal to a value such as 3 mm, 2 mm, 1 mm, 0.8 mm, 0.7 mm, 0.6 mm, 0.5 mm, and the like. The cross-sectional dimension may be the diameter of a circle, the dimension of the largest portion, or the dimension of the smallest portion.

In some embodiments, the light source may be fixed relative to the flow channel. In some embodiments, the photodetector may be fixed relative to the flow channel. In some embodiments, the flow channel or the member including the flow channel may be configured to be detachable from the light source and the photodetector.

The volume of the flow channel may be a volume of a portion in which the optical path is defined, or may include a volume of a portion other than a portion in which the optical path is defined, such as a liquid introduction path or a discharge portion. The volume of the flow channel may be smaller than or equal to 100 *µ*L, 50 *µ*L, 20 *µ*L, 15 *µ*L, 10 *µ*L, 9 *µ*L, 8 *µ*L, 7 *µ*L, 6 *µ*L, 5 *µ*L, 4 *µ*L, 3 *µ*L, 2 *µ*L, 1 *µ*L, 0.9 *µ*L*,* 0.8 *µ*L*,* 0.7 *µ*L*,* 0.6 *µ*L*,* 0.5 *µ*L*,* and the like. The volume of the flow channel may be greater than or equal to 0.01 *µ*L, 0.05 *µ*L, 0.07 *µ*L, 0.1 *µ*L, 0.2 *µ*L, 0.3 *µ*L, 0.4 *µ*L, 0.5 *µ*L, 0.6 *µ*L, 0.7 *µ*L, 0.8 *µ*L, 0.9 *µ*L, 1 *µ*L*,* and the like.

The cross section of the flow channel in which the optical path is defined may be circular, elliptical, a closed figure defined by a curve, or a square, a rectangular parallelepiped, or other polygons.

The length of the optical path in the flow channel may be greater than or equal to a value such as 2 mm, 3 mm, 4 mm, 5 mm, 7 mm, 10 mm, 12 mm, 15 mm, 20 mm, 25 mm, 30 mm, and the like. The length of the optical path in the micro flow channel may be less than or equal to a value such as 100 mm, 90 mm, 80 mm, 70 mm, 60 mm, 50 mm, 40 mm, 30 mm, or the like.

In some embodiments, the flow channel may have an inlet for introducing a solution and an outlet for discharging the solution. By having an inlet and an outlet, it is possible to substantially avoid, for example, air or air bubbles from remaining in the flow channel at the time of introducing the solution, and to facilitate replacement of the solution. In some embodiments, the inlet and the outlet may be the same and may have an air hole. In some embodiments, the flow channel of the portion where the optical path is defined may have an inlet and an outlet of the solution in the flow channel at both ends.

In some embodiments, some or all of the inner walls of the flow channel may be hydrophilic. It is expected that the hydrophilic micro flow channel reduces a non-uniform distribution when the solution is introduced into the micro flow channel and avoids the generation of bubbles.

In some embodiments, the photodetector may include a photodiode. In some embodiments, the photodetector may include a device for measuring brightness. The photodetector may be a CCD, a photomultiplier, a scintillator, or the like. In some embodiments, the photodetector may be a spectrometer.

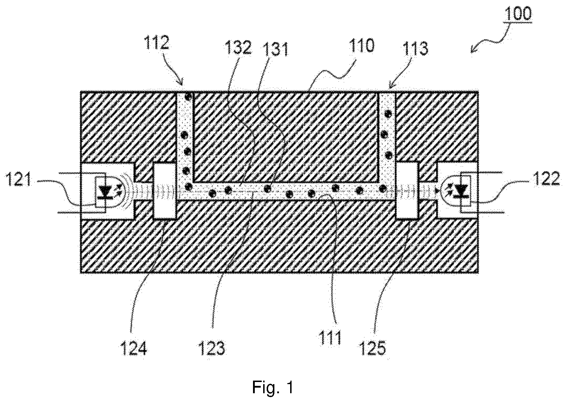

FIG. 1 shows an optical measurement apparatus 100 according to one embodiment. The main body 110 has a U-shaped flow channel 111. A portion including an optical path 123 and an inlet 112 and an outlet 113 for introducing and discharging a solution into the optical path 123 portion thereof are provided. The portion including the optical path 123 is a micro space at the microliter level, and is formed to be elongated along the optical path 123. Compared to the size of the cross-sectional direction of the optical path 123 (which may be defined by the cross-sectional area, diameter, maximum diameter, minimum diameter, average diameter, or the like), the length of the optical path 123 direction is taken sufficiently long. Thereby, while the flow channel 111 has a minute volume, it allows the measurement target substance and light to sufficiently react absorbance with light, such as sufficient.

A solution 132 including a measurement target substance 131 is introduced from the inlet 112. From the outlet 113, the solution 132 can be discharged from the flow channel 111 by suction or pressurization from the inlet 112. Outside both ends of the optical path 123 in the flow channel 111, a light source 121 and a photodetector 122 are disposed. In FIG. 1, they are both shown as a light emitting diode and a photodiode by way of example. The main body 110 of FIG. 1 is formed of a non-transparent material. Thereby, the external light can be blocked. On the other hand, in order to propagate the light emitted from the light source 121 into the flow channel, a transparent light entrance window 124 is disposed. Further, on the opposite side with respect to the flow channel having the light entrance window 124 and the optical path 123, the light exit window 125 formed of a transparent material similarly to the light entrance window 124 is disposed. The light entrance window 124 and the light exit window 125 pass light from the outside of the flow channel 111 while sealing the flow channel 111 from the outside so that the solution 132 does not leak out of the flow channel 111, and the measurement can be performed with sufficient accuracy on the absorbance or the like of the measurement target substance 131 in the solution 132 contained in the flow channel 111.

In some embodiments, the solution inlet and outlet may be fluidly coupled to the flow channel at an entrance end (one end) and an exit end (the other end). In some embodiments, the inlet and the outlet may be fluidly coupled at both ends of the optical path in the flow channel. In some embodiments, the inlet and the outlet may be arranged such that no dead space is substantially formed in the flow channel or such that a dead space is avoided. Thus, the arrangement of the inlet and the outlet substantially avoids air bubbles from forming in the flow channel and affecting the optical measurement, such as during the introduction of the solution.

In some embodiments, the apparatus may further include a shield configured to prevent light from other than the light source from being substantially detected by the photodetector.

In some embodiments, the apparatus or the flow channel may be disposed in a shielded box. In some embodiments, it may be a flow channel member having a shielding function.

In some embodiments, the member forming the flow channel may include a shielding material.

In some embodiments, the light source may have a plurality of peak wavelengths. In some embodiments, the light source may include two or more light sources. The plurality of light sources may generate electromagnetic waves or light of a plurality of wavelengths or a plurality of wavelength regions different from each other. In some embodiments, the peak wavelength (the wavelength at which the output value is the highest among the ranges or spectral distributions of light emission) of a light source may be different from at least one of the plurality of light sources. In some embodiments, one peak wavelength (or frequency) may be a wavelength (or frequency) characteristic of the absorption characteristic of the measurement target substance or a wavelength in its vicinity, and the other peak wavelength may be a wavelength (or frequency) having no influence or less influence of the absorption characteristic. In some embodiments, the light source may include a plurality of light sources each having a different peak wavelength.

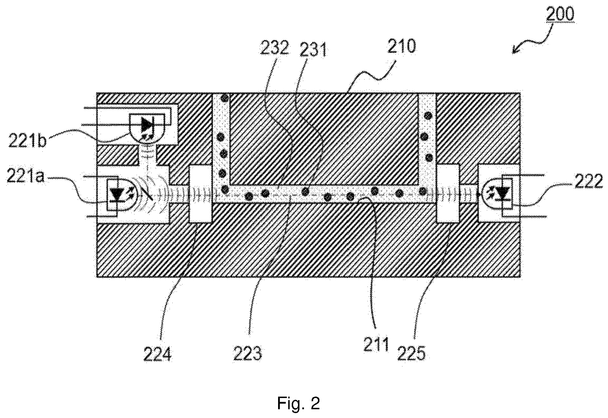

FIG. 2 schematically shows an optical measurement apparatus 200 according to one embodiment having two light sources 221a,221b. A flow channel 211 is formed in a housing 210, and two light sources 221a,221b are fixed thereto. The two light sources 221a,221b emit light of different wavelengths or peak wavelengths. In the optical measurement apparatus 200 of FIG. 2, the first light source 221a is configured to be introduced into the flow channel 211 straight through the light entrance window 224. The light from the second light source 221b is refracted by a half mirror 226 provided on the optical path from the first light source 221a to the light entrance window 224, and propagates along the optical path 223 of the light from the first light source 221a in the flow channel 211. Thereby, both the light from the light sources 221a,221b propagate in substantially the same optical path 223 and react with the solution 231 and the measurement target substance 232 under substantially the same incident conditions. The light after the reaction passes the light exit window 225 and enters the photodetector 222.

In FIG. 2, only one photodetector 222 is disposed, it is difficult to determine the luminances at two wavelengths, respectively, from the intensities of two lights that are incident at the same time. Therefore, as an example, the first light source 221a and the second light source 221b may be made to emit light alternately, or emit light in such a manner that the emission times do not overlap (the control mechanism is not shown). Thereby, by the luminance measurement of the photodetector 222 within each emission time, the respective absorbance and the like can be determined.

In some embodiments, the apparatus may be configured to introduce light from only one of the plurality of light sources into the optical path. In some embodiments, the apparatus may further comprise a light source control mechanism for selecting a light source in such a manner. In some embodiments, the apparatus may have a mechanism or a light source control mechanism that passes only the light used for the measurement and blocks the light not used for the measurement. In some embodiments, it may have a drive controller of the light source for driving only the light source for emitting the peak wavelength used for measurement and not driving the other light sources. In some embodiments, the apparatus may include a drive controller for controlling the light emission of the plurality of light sources. In some embodiments, the light source control mechanism may include a drive controller for controlling the emission of the plurality of light sources.

In some embodiments, the apparatus may have an additional photodetector (which may alternatively be referred to as second photodetector, adjustment photodetector, or the like). The adjustment photodetector can be used to observe or measure the emission of the light source to control the emission of the light source. Such a photodetector may be disposed outside the light path of the light emitted by the light source, and may be configured to receive and measure a portion of the light emitted by the light source.

In some embodiments, the apparatus may include a controller that receives information (for example, intensity or brightness of received light) regarding the light characteristics from the adjustment photodetector and controls the light emission of the light source. The controller may control the amount of light emitted by the light source to be substantially constant over time.

In some embodiments, the apparatus may be configured to regulate the temperature of the solution in the flow channel. In some embodiments, the apparatus may further comprise a temperature controller or adjuster that regulates the temperature of the solution in the flow channel.

In some embodiments, the temperature within the flow channel may be controlled by regulating or keeping constant the temperature outside of the apparatus or outside of the members forming the micro flow channels. The temperature of the atmosphere of the components forming the apparatus or the micro flow channel may be regulated, and the temperature of the portions or blocks in contact therewith may be regulated.

The temperature in the micro flow channel or near the micro flow channel may be measured by a temperature sensor. Temperature control may be performed using measured temperature information (temperature, changes in temperature, calculations based on multiple temperature measurements, and the like).

In some embodiments, the apparatus may include a heater. The heater may be disposed in the vicinity of the micro flow channel. The heater may be configured to control the temperature of the solution.

In some embodiments, the member forming the flow channel may have a higher heat capacity than the solution in the flow channel. If the heat capacity of the member around the solution is large enough, the temperature of the solution will become substantially the temperature of the same member.

In some embodiments, all or at least a portion of the member forming the flow channel may be formed of a material having a high thermal conductivity, may be formed of, for example, a metal such as copper, iron, stainless steel and the like or an alloy.

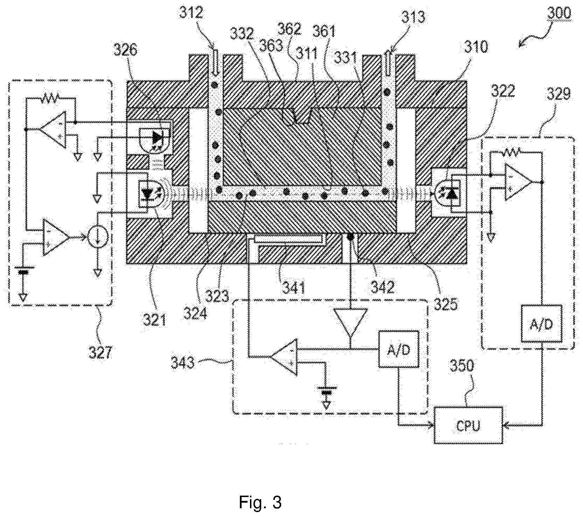

FIG. 3 shows an optical measurement apparatus 300 according to one embodiment. The optical measurement apparatus 300 of FIG. 3 comprises various control mechanisms.

The optical measurement apparatus 300 of FIG. 3 has an adjustment photodetector 326 for measuring the amount of light emission of the light source 321. In FIG. 3, the light source 321 and the adjustment photodetector 326 have a photodiode. The adjustment photodetector 326 is disposed so as to receive light exiting substantially laterally from the light source 321 with respect to the direction of the optical path 323. With such an arrangement, the adjustment photodetector 326 does not block the light to the optical path 323, also a sufficient amount of light for control can be obtained. The brightness or emission intensity of the light source 321 may change temporally by such heat generated by itself by the light emission time and light emission. The adjustment photodetector 326 can capture a temporal change in the amount of light emission of the light source 321. In FIG. 3, the controller 327 of the light source 321 is disposed to adjust the current flowing through the light source 321, so that the output current from the adjustment photodetector 326 is constant.

In FIG. 3, the temperature sensor 342 can measure the temperature of the housing 361 near the flow channel 311. Further, a heater 341 is provided in the vicinity of the flow channel 311 by the temperature sensor 342, so that the solution 332 including the measurement target substance 331 can be heated through a part of the housing 361. The temperature controller 343 is configured to receive information from the temperature sensor 342 and supply the necessary power to the heater 341.

Photodetector 322 has a photodiode in FIG. 3. The output of the photodetector 322 (current) is converted into a voltage at the photodetector output circuit 329, and the voltage value is sent to the processing circuit (CPU) 350. From the output of the photodetector 322, the intensity of light received by the photodetector 322 can be determined. The processing circuit 350 can compare the output signal of the photodetector 322 with the intensity of light in the absence of the measurement target substance 331, to determine the absorbance or the like. The processing circuit 350 can acquire temperature information of the solution 332 by the temperature sensor 342, and perform correction of absorbance calculation based on the temperature information.

In FIG. 3, the mechanical configuration is largely divided into three parts. That is, these are three parts: the main body 310 for fixing a light source 321, an adjustment photodetector 326, a photodetector 322, a temperature sensor 342, and a heater 341; a housing constituting the U-shaped flow channel (flow channel housing) 361; and a fixing portion 363 securing the inlet 312 and the outlet 313 as well as fixing the flow channel housing 361 to the main body 310 accurately. In FIG. 3, both the light window 324 and the light exit window 325 are fixed to the flow channel housing 361. Thus, an optical system such as a light source 321 and electrical systems such as a temperature control device are fixed to the main body 310. In contrast, the flow channel housing 361 and the fixing portion 362 for passing the measurement target substance 331 and the solution 332 including a biomaterial or a chemical substance and the like are configured to be detachable with respect to the main body 310. In some embodiments, the positional relationship between the optical path 323 and the flow channel 311 is not important for measurement of absorbance or the like. On the other hand, it is important in some embodiments. In such a case, the main body 310 and the flow channel housing 361 or even the fixing portion 362 exemplarily shown in FIG. 3 can enhance the positioning accuracy of the flow channel 311 and the optical path 323 by the fitting 363, and can ensure the fluid coupling of the inlet port 312 and the outlet port 313 to the flow channel 311.

In some embodiments, the apparatus may have a plurality of flow channels. Each flow channel may define a corresponding optical path.

In some embodiments, the apparatus may have a first flow channel and a second flow channel, wherein the first flow channel includes or defines a first optical path and the second flow channel includes or defines a second optical path. The first optical path and the second optical path are optical paths in which light emitted from the light source propagates. In some embodiments, the apparatus may include a first photodetector for detecting light that has passed through the flow channel and a second photodetector for detecting light that has passed through the second flow channel.

In some embodiments, the light source may include a plurality of light sources. In some embodiments, each of the different light sources may correspond to a respective flow channel. In some embodiments, the light source includes a first light source and a second light source, and the light path may include a first light path through which light emitted from the first light source propagates and a second light path through which light emitted from the second light source propagates. In some embodiments, the flow channel may include a first flow channel extending along the first optical path and a second flow channel extending along the second optical path. In some embodiments, the photodetector may include a first photodetector for detecting light that has passed through the first flow channel and a second photodetector for detecting light that has passed through the second flow channel.

In some embodiments, the first light source and the second light source may be the same light source. Light emitted from the same light source may be branched into a first optical path and a second optical path, respectively, using a beam splitter or the like.

In the flow channel housing 361 shown in FIG. 3, the flow channel portion including the optical path 322 is formed as a through hole. The light entrance window 324 and the light exit window 325 are attached to side surfaces of the flow channel housing 361, and covers both ends of the through hole. Such transparent light windows enable light to enter or exit efficiently with respect to the flow channel, as well as enable the low-cost manufacture of optical cells.

In some embodiments, the apparatus may further include a container to contain the solution. The container may be fluidly connected to the flow channel. The apparatus may be configured to deliver a container contained in the container to the flow channel. In some embodiments, the container may contain a solution to be mixed with the measurement target substance. Such a container may be configured to receive a solution including a measurement target substance with respect to a container storing in advance, and mix them inside the container. The container may be configured to deliver a further mixed solution to the flow channel.

In some embodiments, the container containing the solution may include a plurality of containers. In some embodiments, each of the plurality of flow channels may be arranged with a container containing a solution in fluid connection. In some embodiments, the apparatus may comprise a first container configured to be fluidly coupled to the first flow channel and a second container configured to be fluidly coupled to the second flow channel. In some embodiments, one of the first container and the second container may have an inlet (container inlet) for introducing the measurement target substance. In some embodiments, at least one of the first container and the second container may be configured to mix the already contained solution (mixed solution) with the introduced measurement target substance or the solution including the measurement target substance (original liquid or diluted solution of the original liquid) inside thereof.

In some embodiments, the apparatus may include a mixing mechanism for mixing a solution including a measurement target substance and a mixed solution. The mixing mechanism may be a mechanism for moving the whole such as shaking the container for mixing, and may be a mechanism for mechanically deforming a part or the whole of the container. The mixing mechanism may be disposed directly with respect to the container for mixing or in the vicinity thereof. The mixing mechanism may be a flow channel. Flow channel as a mixing mechanism may be configured to have a plurality of flow channels, may be curved linearly bent or curved, may have a diameter or cross-sectional area or cross-sectional shape which changes as it proceeds in the direction of the flow.

In some embodiments, the apparatus has a plurality of flow channels, wherein at least one flow channel is a flow channel containing a solution to be measured, and at least another flow channels may have a flow channel for performing a reference measurement or a flow channel for reference measurement. In some embodiments, the apparatus may further include a reference measurement flow channel including at least a portion of the reference measurement optical path and extending along the reference measurement optical path. In some embodiments, the reference measurement flow channel may be sealed. In some embodiments, the reference measurement flow channel may include an inlet and an outlet.

In some embodiments, the apparatus may further include a reference measurement optical system configured to propagate light emitted from the light emitter along a reference measurement optical path in the reference measurement flow channel and receive the light at the photodetector. The reference measurement optical system may include a beam splitter.

In some embodiments, it may include a reference measurement light source for propagating light along a reference measurement optical path in the reference measurement flow channel, and a reference measurement photodetector for detecting light emitted from the light source and having propagated in the reference measurement flow channel along the reference measurement optical path. In some embodiments, the apparatus may include a light source and a photodetector for a reference measurement, apart from the main optical system for measurement.

In some embodiments, a reference solution may already be filled in the reference measurement flow channel.

In some embodiments, the reference solution may be purified water for example, for measurement of a measurement target substance. In some embodiments, the reference measurement flow channel may be filled with air. In some embodiments, it may be a mixture of purified water and a luminescent material (for example, BCP) solution. The reference solution may be other solutions.

In some embodiments, the reference solution may be filled in the reference flow channel at the time of shipment or prior to shipment of the apparatus. In some embodiments, the reference solution may be stored in a reference solution container in the apparatus and configured to be mixed with the solution at a necessary timing.

In some embodiments, the reference measurement flow channel may have a substantially similar configuration as the flow channel. Thereby, for example, the accuracy of the reference measurement can be increased. By performing an optical measurement substantially the same as that for the target substance with respect to the reference solution in the flow channel for reference measurement, calibration of the absorbance measurement can be performed with relatively high accuracy.

In some embodiments, a chromogenic substance (hereinafter, including an indicator, a chromogenic reagent, a coloring reagent, and an absorbance standard substance) may be bonded to a measurement target substance. In some embodiments, the absorbance of the chromogenic substance (or the optical characteristics associated with the absorbance or the change thereof) may be measured, and the concentration of the measurement target substance may be specified from the same absorbance or the like. In some embodiments, a dye-binding method may be used.

In some embodiments, the apparatus may have a chromogenic substance container comprising a chromogenic substance. In some embodiments, the apparatus may have an inlet for the measurement target substance and may have a mechanism for mixing the measurement target substance introduced from the inlet and the chromogenic substance. In some embodiments, the apparatus may have a mechanism for delivering a mixed solution of a measurement target substance and a chromogenic substance to a flow channel.

In certain embodiments, the measurement target substance may comprise albumin. The albumin contained in the measurement target may be albumin in blood or albumin in saliva or tear. In some embodiments, the chromogenic substance may be methyl violet, litmus, promocresol green, and may be methyl orange, bromocresol purple, phenol red. The chromogenic substance, in other examples, may be iodine which reacts with starch, ninhydrin which reacts with an amine or an amino acid, silver nitrate which reacts with chloride ions, and the like. The chromogenic substance is not limited to the above examples.

In some embodiments, it may be configured to mix an oxidizing agent with a measurement target substance or a solution including a measurement target substance. For example, an oxidizing agent may be mixed into tears. In some embodiments, the apparatus may have an oxidant container containing an oxidizing agent. Biomolecules in which there are oxidized and reduced forms may differ in the properties of modifications, such as chromogenic substances, between types. Therefore, exemplarily, by oxidizing before binding a chromogenic substance or the like, the amount of binding with the chromogenic substance can be reduced and furthermore measurement errors between biomolecules and between measurements can be reduced.

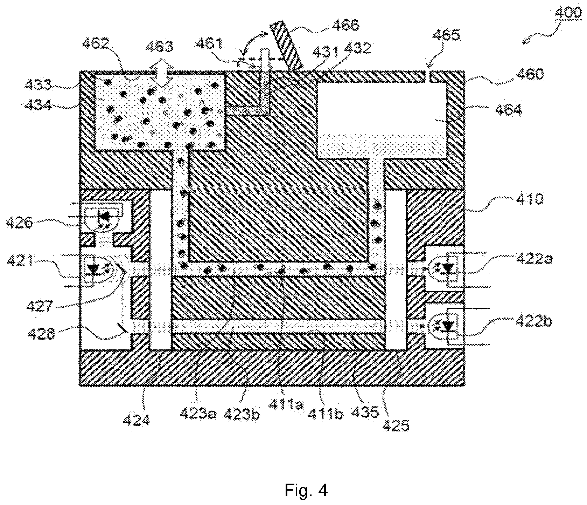

FIG. 4 shows an optical measurement apparatus 400 according to one embodiment. The optical measurement apparatus 400 shown in FIG. 4 includes two flow channels 411a,411b in which optical paths 423a,423b are defined, a solution to be mixed with a original liquid 432 including a measurement target substance 431 (mixed solution, measurement solution), a container 462 for mixing a measurement solution 434, and a mixing mechanism 463 (details are not shown).

The flow channel member 460 forming the flow channel system of the apparatus 400 has an inlet 461. From this inlet 461, a solution 432 including an original liquid or a measurement target substance 431 is introduced. An openable and closable sealing lid 466 is connected to the inlet 461 in order to prevent backflow or leakage of the solution and penetration of impurities into the solution or the flow channel.

The flow channel member 460 has a mixing container 462, and a measurement solution 434 including a chromogenic substance 433 having a property of binding to the measurement target substance 431 is stored in advance. The introduced original liquid 432 is introduced into this mixing container 462, and the chromogenic substance 433 is bonded or adhered to the measurement target substance 431. The mixing container 462 is provided with a mixing mechanism 463 (details of which are not shown). The mixing mechanism 463 pushes the elastic membrane above the mixing container 462 up and down, thereby changing the volume or shape of the mixing container 462. Thereby, exemplarily, a reaction such as binding between the introduced measurement target substance 431 and the chromogenic substance 433 can be promoted and uniformly performed. A solution including the measurement target substance 431 to which the chromogenic substance 433 is bound by mixing is introduced into the first micro flow channel 411a from one end thereof.

The micro flow channel 411a for measuring the measurement target substance 431 is fluidically connected to a reservoir 464 at the other end thereof. For example, the solution which has passed through the flow channel 411a from the container 462 by pressing and has filled the micro flow channel 411a needs a place to escape. The storage container 464 may have its function. In addition, the reservoir 464 having a sufficient volume is effective when the solution reciprocates not only in one direction but also in the flow channel 411a. The reservoir 464 of FIG. 4 has an air hole 465 leading to the fluid member 460 or to the outside of the apparatus 400 to allow air to enter and exit from the air hole in response to the movement of the solution, thereby allowing smooth movement of the solution in the flow channel 411a.

On the other hand, the second micro flow channel 411b is a flow channel for reference measurement. The space of the second micro flow channel 411b is sealed between the light entrance window 424 and the light exit window 425. The sealed space is previously filled with a reference measurement solution 435. The reference measurement solution 435 of FIG. 4 is the same solution as the measurement solution 434 previously filled in the container 462.

The apparatus 400 has a single light source 421 fixed to the main body 410, the amount of light is controlled using the adjustment photodetector 426. The light emitted from the light source 421 is first separated into two by the beam splitter 427 so as to be guided to the optical paths 423a,423b of the two flow channels 411a,411b, respectively. One of them directly travels straight, passes through the light entrance window 424, and travels along the optical path 423a in the micro flow channel 411a. The other light is further refracted by mirror 428 and travels along the optical path 423b in the micro flow channel 411b past the light entrance window 424.

At the other ends of the micro flow channels 411a,411b, photodetectors 422a,422b are fixed to the main body 410, respectively, the intensity of the emitted light can be measured. The internal shapes, dimensions, and inner wall properties of the micro flow channel 411a,411b are substantially the same, and the internal solutions occupying the respective flow channels 411a,411b are also the same. Therefore, by calculating the difference between the intensity at the photodetector 422a and the intensity at the photodetector 422b, the measurement error due to the solution or the wall of the flow channel can be arithmetically removed, and the absorbance or the like corresponding to the chromogenic substance 433 that was in the first micro flow channel 411a at the time of measurement can be more accurately obtained.

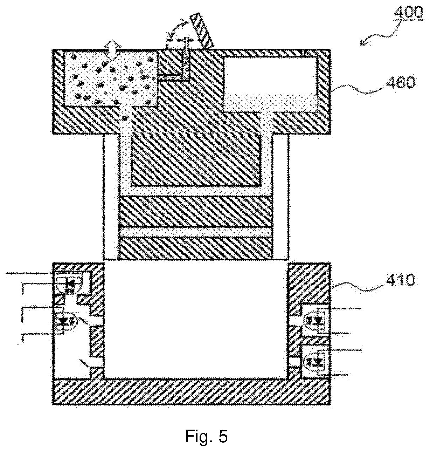

As shown in FIG. 5, the flow channel member 460 including the flow channel system is detachably attached to the main body 410 including the optical system. In some embodiments, the optical paths 423a,423b of the optical system are positioned with relatively high positioning accuracy with respect to the micro flow channels 411a,411b in the flow channel member 460.

In some embodiments, the absorbance measurement apparatus may include a plurality of absorbance measurement devices, may be combined with an apparatus of other measurement methods, may be configured to be connected or combined with other measurement devices or measurement apparatuses, and may be a combined system.

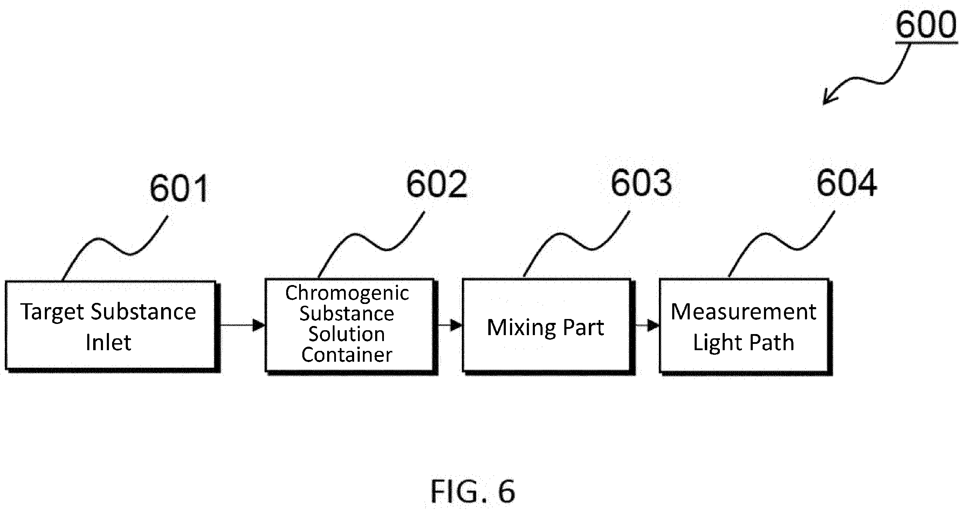

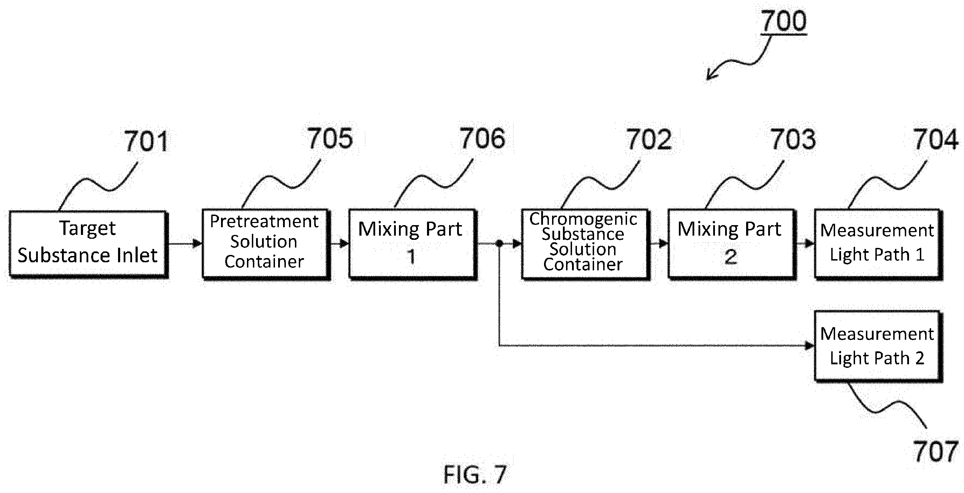

FIG. 6 shows a block diagram of the configuration of an optical measurement apparatus 600 according to one embodiment. A solution including a measurement target substance is introduced into the apparatus from an inlet 601 and mixed with a solution of a chromogenic substance in a container 602 containing a chromogenic substance. In FIG. 7, to better mix these, a mixing portion 603 is provided. The measurement target substance and the chromogenic substance can be sufficiently reacted and bound by the mixing portion 603. The solution including the target substance to which the chromogenic substance is bound is sent to the measurement light path 604. In the measurement light path 604, an optical measurement is substantially performed on the chromogenic substance.

In some embodiments, the measurement apparatus may have a pretreatment solution or a container containing a pretreatment solution for performing pretreatment on a measurement target substance introduced into the apparatus or a solution including a measurement target substance (including an original liquid, a dilution solution thereof, and the like)
In some embodiments, the measurement apparatus may have a plurality of measurement optical paths. In some embodiments, the measurement apparatus may have at least two measurement optical paths, one of which may be used as an optical path for the main measurement, and the other measurement optical path may be used to measure a comparison or a baseline (or background).

FIG. 7 shows a block diagram of the configuration of a measurement apparatus 700 according to one embodiment. A solution including a measurement target substance is introduced into the apparatus from an inlet 701 and sent to a container 705 containing a pretreatment solution. In the same container, the measurement target substance is mixed with the pretreatment solution. In FIG. 7, a first mixing portion 706 is provided for better mixing. By the first mixing portion 706 the measurement target substance and the pretreatment solution can be sufficiently bound with each other. In some embodiments, the pretreatment solution may be an oxidizing agent. Next, the solution including the pretreated measurement target substance is divided into a flow channel in which it reacts with the chromogenic substance and a flow channel in which it does not react with the chromogenic substance.

One of the divided solutions is introduced into a container 702 containing a chromogenic substance solution, where the measurement target substance after the pretreatment and the solution of the chromogenic substance are mixed. In FIG. 7, a second mixing portion 703 is provided to better mix these. By the second mixing portion 703, the measurement target substance and the chromogenic substance can sufficiently react and combine with each other. The solution including the target substance to which the chromogenic substance is bound is sent to a first measurement light path 704. In the first measurement light path 704, an optical measurement is substantially performed on the chromogenic substance.

The other of the divided solution is sent to a second measurement path 707 without being bound with the chromogenic substance. In the second measurement light path 707, the optical influence by the pretreatment solution can be measured substantially without the influence of the chromogenic substance. In other words, in the second measurement optical path 707, the baseline is measured.

The difference between the information obtained in the main measurement in the first measurement light path 704 and the information obtained in the baseline measurement of the second measurement light path 707 can be taken as a substantial optical measurement result for the chromogenic substance.

In the measurement apparatus 700 as shown in FIG. 7, the liquid feed is performed in one direction toward the measurement light path from the inlet. Thus, by way of example, the liquid feed mechanism is simplified, and the apparatus can be reduced in size accordingly. In addition, for example, by feeding the fluid in one direction in the flow channel by pushing from the inlet by a pump or the like, that is, by feeding the fluid under a positive pressure, the generation of bubbles or air bubbles can be prevented.

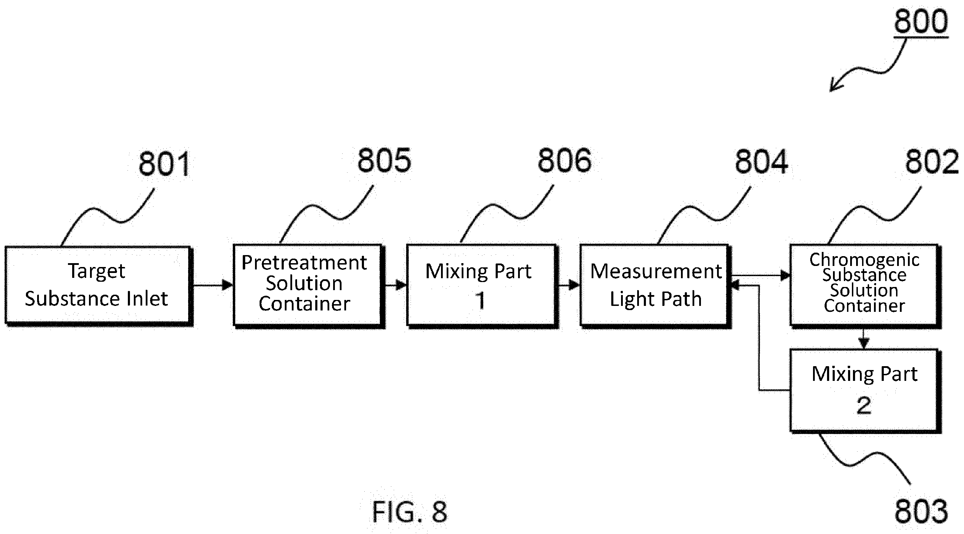

FIG. 8 shows a block diagram of the configuration of a measurement apparatus 800 according to one embodiment. A solution including a measurement target substance is introduced into the apparatus from an inlet 801 and sent to a container 805 containing a pretreatment solution. In the same container, the measurement target substance is mixed with the pretreatment solution. In FIG. 8, a first mixing portion 806 is provided for better mixing. By the first mixing portion 806 the measurement target substance and the pretreatment solution can be sufficiently bound with each other. In some embodiments, the pretreatment solution may be an oxidizing agent. Next, an optical measurement is performed on the solution including the pretreated measurement target substance in the measurement light path 804. Thereby a baseline measurement is performed.

Thereafter, the solution is introduced into the container 802 containing the chromogenic substance solution, where the measurement target substance after the pretreatment and the solution of the chromogenic substance are mixed. In FIG. 8, a second mixing portion 803 is provided to better mix these. By the second mixing portion 803, the measurement target substance and the chromogenic substance can sufficiently react and bind with each other. The solution including the target substance to which the chromogenic substance is bound is again sent to the measurement light path 804. In the measurement light path 804, an optical measurement is substantially performed on the chromogenic substance. In the measurement light path 804, an optical measurement is substantially performed on the chromogenic substance. Thereby, the main measurement is performed.

Then, the difference between the information obtained in the baseline measurement performed first and the information obtained in the baseline measurement performed next can be taken as a substantial optical measurement result for the chromogenic substance.

In the measurement apparatus, as shown in FIG. 8, two optical measurements can be performed in one optical path. Thus, by way of example, the number of measurement light paths can be reduced, the size of the corresponding apparatus can be reduced, and the cost by reducing the number of parts can be lowered.

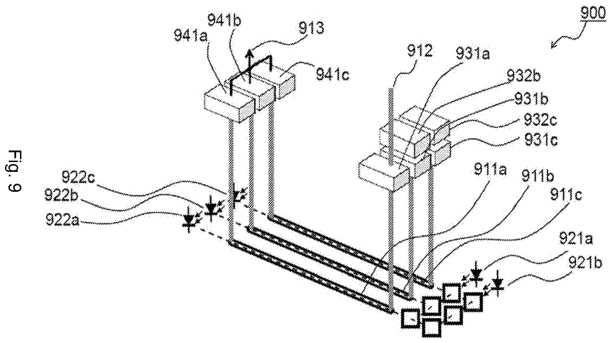

FIG. 9 shows a perspective view schematically showing an optical system and a flow channel system of a measurement apparatus 900 according to one embodiment. Housing, mechanical parts such as mixing mechanism and liquid feed mechanism, control system, and the like are not shown. The measurement apparatus 900 shown in FIG. 9 has flow channels 911a,911b,911c including three measurement optical paths, which are used for main measurement, buffer measurement, and reference measurement, respectively. Two solutions, one for pretreatment and the other for chromogenic substances, which are mixed or reacted with the measurement target substance, are prepared with the flow channel sandwiched between them. That is, the containers 931a,931b,931c containing the solution including the pretreatment solution are disposed on the upstream side of the flow channels 911a,911b,911c, respectively, and the containers 941a,941b,941c containing the solution including the chromogenic substance are disposed on the downstream side of the flow channels 911a,911b,911c, respectively. Two light sources 921a,921b are arranged, the light emitted from each passes through the flow channels 911a,911b,911c are received by the photodetectors 922a,922b,922c disposed opposite the respective flow channels.

First, the flow channel 911a for main measurement will be described. A solution including a measurement target substance (or an original liquid such as a diluted solution thereof or the like) is introduced into the apparatus 900 from the inlet 912. The solution including the introduced measurement target substance enters the container 931a containing the pretreatment liquid, and is mixed, and the pretreatment is performed. The solution after the pretreatment is introduced into the measurement flow channel 911a, and fills the flow channel portion 911a in which the optical path is defined. Thereafter, measurements are performed in two wavelength regions emitted from two light sources 921a,921b. The light sources may be turned on sequentially or alternately so that light from one light source passes through the flow channel at a point of time.

After the measurement of the solution after the pretreatment is completed, the solution is pushed and introduced into the container 941a containing the solution of the chromogenic substance. The measurement substance and the chromogenic substance are bound. The pressure as the solution is extruded can be released to the outside through the air vent 913. When the measurement substance and the chromogenic substance are sufficiently bound, the solution is sent in the reverse direction and introduced into the flow channel 911a again. Then, the measurement is performed on the chromogenic substance.

Next, a buffer measurement flow channel 911b and a reference solution measurement flow channel 911c will be described. In either case, the measurement target substance is not introduced. The solutions contained in the buffer container 932b and the reference solution 932c are introduced into the containers 931b,931c containing the pretreatment liquid, respectively, and mixed, and the pretreatment is performed.

The buffer solution is a solution not including the measurement target substance. Thereby, the measurement corresponding to the concentration zero can be performed.

The solution for reference measurement is a solution including the same or equivalent measurement target substance at a known concentration. In some embodiments, a measurement target substance having a concentration corresponding to a concentration region of a measurement target substance assumed in the main measurement may be contained in the solution for reference measurement. In some embodiments, a measurement target substance having a concentration sufficiently different from the concentration region of the measurement target substance assumed in the main measurement may be contained in the solution for reference measurement.

Thereafter, the operation is the same as that of the main measurement flow channel. In other words, the solution after the pretreatment is introduced into the measurement flow channels 911b,911c, respectively, and fills the flow channel portions 911b,911c in which the optical path is defined. Thereafter, measurements are performed in the two wavelength regions emitted from the two light sources 921a,921b. After the measurement of the solution after the pretreatment is completed, the solution is pushed and introduced into the containers 941b,941c containing the solution of the chromogenic substance. The measurement substance and the chromogenic substance combine. The pressure as the solution is extruded can be released to the outside through the air vent 913. After a period of time similar to the binding of the measurement substance and the chromogenic substance in the flow channel for measurement, the solution is sent in the reverse direction and introduced into the flow channels 911b,911c again. Then, the same optical measurement is performed.

The measurement results (information) obtained from these six measurements can be calculated as follows to calculate the concentration of the chromogenic substance to be determined.

In each flow channel, post-pretreatment measurements (baseline measurements) provide a baseline. The information obtained in the measurement of the target substance to which the chromogenic substance (the main measurement) is bound is the sum of the information of the desired chromogenic substance and the information based on the baseline. Therefore, the measurement information of the desired substance is the information obtained by subtracting the information obtained by baseline measurement from the information obtained by the main measurement.

The difference may be taken if the baseline measurement is V1 and the main measurement is V2. That is,
ΔV=V2 -V1

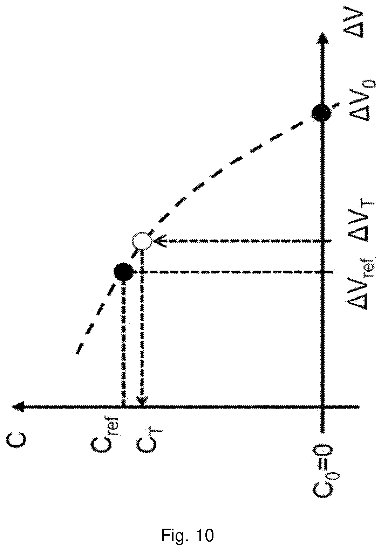

For this ΔV, the value ΔVb (or ΔV0) from the measurement value in the buffer flow channel 911b corresponds to zero concentration (C=0 or C0) of the target substance. The value ΔVc (or ΔVref) from the measured value in the buffer flow channel 911c corresponds to the reference concentration (C=Cref) of the target substance. Since these four values, {ΔV0, C0}{ΔVref, Cref} correspond to two points in the space of {measurement V (or ΔV), concentration C}, the relation of {measurement V, concentration C} can be represented by a straight line passing through these two points, or other one-to-one corresponding curves. Based on this relationship, the concentration (CT) in the flow channel 911a of the chromogenic substance to be determined can be determined from the measured value ΔVa (ΔVT) in the flow channel 911a. See FIG. 10.

According to the present disclosure, an optical measurement apparatus (such as a GA level measurement apparatus) having a footprint (or occupied area, device size) of several centimeters by several centimeters or smaller can be manufactured on the basis of a micro flow channel. The footprint may be smaller than or equal to a value such as 25 cm², 20 cm², 16 cm², 15 cm², 12 cm², 10 cm², 9 cm², 8 cm², 6 cm², and 4 cm².

The photodetector 922a,922b,922c of the measurement apparatus of FIG. 9 is a diode and the output is a current. Thus, the measured value may be a current value. For diodes, the current value is proportional or related to the intensity of the input light. Alternatively, the current value which is a direct output of the diode may be converted to a voltage value, the voltage value may be taken as the measured value. The measured value may be a value related to the output of the photodetector, and is not limited to the above example.

The measurement substance may be an inorganic substance and may be an organic substance. In some embodiments, the measurement target substance may be a biomolecule. In some embodiments, the measurement target substance may be a protein included in a body fluid. In some embodiments, the solution (or diluent, original liquid) including the measurement target substance may be a bodily fluid. The "body fluid" may be blood, serum, plasma, lymph fluid, tissue fluids such as interstitial fluid, intercellular fluid, interstitial fluid, and the like, and may be body cavity fluid, serosal fluid, pleural fluid, ascites fluid, pericardial fluid, cerebrospinal fluid, joint fluid (synovial fluid), and aqueous humor of the eye (aqueous). The body fluid may be digestive fluid such as saliva, gastric juice, bile, pancreatic juice, intestinal fluid, and the like, and may be sweat, tears, nasal mucus, urine, semen, vaginal fluid, amniotic fluid, milk, and the like. The body fluid may be a body fluid of an animal and may be a body fluid of a human. The "body fluid" may be a solution. The solution may include a buffer solution such as phosphate buffered saline (PBS) or N-tris(hydroxymethyl)methyl-2-aminoethanesulfonic acid buffer (TES) including a measurement target substance. The solution is not particularly limited as long as the measurement target substance is included.

The solution may include a measurement target substance. For example, the solution may be tear and the measurement target substance may be albumin or glycoalbumin included in tears. Alternatively, the measurement target substance may be albumin, glycoalbumin, hemoglobin, glycohemoglobin in blood or serum, may be albumin, glycoalbumin in interstitial fluid, may be albumin, glycoalbumin in tears, and may be albumin, glycoalbumin in urine, or the like.

In some embodiments, the measurement target substance is albumin, and the original liquid may be tear or saliva. In some embodiments, the pretreatment liquid may be an oxidizing agent. In some embodiments, the chromogenic substance may be BCP (bromocresol purple). In some embodiments, the wavelengths of the light sources 921a,921b may be 600 nm and 660 nm or wavelengths in the vicinity thereof, respectively.

In some embodiments, the measurement apparatus may include a plurality of measurement units, each of which is composed of a main measurement flow channel system, a buffer measurement flow channel system, and a reference solution measurement flow channel system, as one measurement unit. In some embodiments, multiple measurement units may perform same or similar measurements multiple times simultaneously, synchronously, at varying times, or asynchronously. In some embodiments, at least two of the plurality of measurement units may perform different measurements.

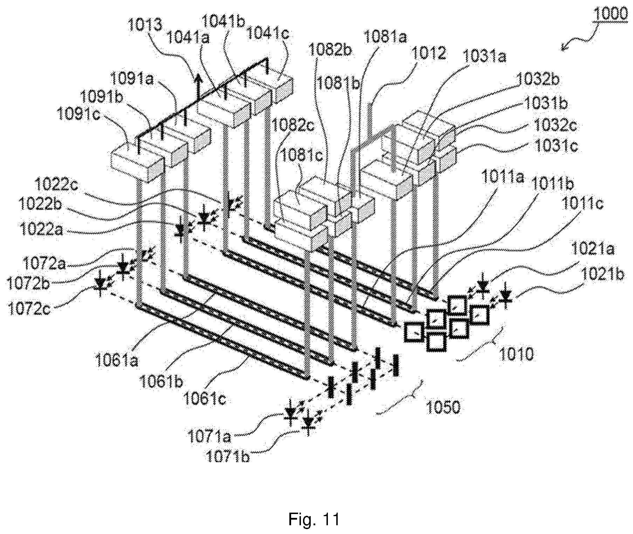

FIG. 11 shows a perspective view schematically showing an optical system and a flow channel system of a measurement apparatus 1000 according to one embodiment. Housing, mechanical parts such as mixing mechanism and liquid feed mechanism, the control system, and the like are not shown. The measurement apparatus shown in FIG. 11 has two measurement units 1010,1050.

The measurement unit 1010 is the same measurement unit as in FIG. 9. That is, the measurement unit 1010 shown in FIG. 11 has flow channels 1011a,1011b,1011c including a measurement optical path, which are used for the main measurement, the buffer measurement, and the reference solution measurement, respectively. Two solutions, one for pretreatment and the other for chromogenic substances, which are mixed or reacted with the measurement target substance, are prepared with the flow channel sandwiched between them. In other words, on the upstream side of the flow channels 1011a,1011b,1011c, containers 1031a,1031b,1031c containing solutions including a pretreatment solution, a buffer container 1032b containing a buffer solution and a reference reference solution 1032c including a reference solution are disposed corresponding to the respective flow channels. On the downstream side of the flow channels 1011a,1011b,1011c, containers 1041a,1041b,1041c containing solutions including a stored chromogenic substance are disposed corresponding to the respective flow channel 1011a,1011b,1011c. Two light sources 1021a,1021b are arranged, and the light emitted from each passes through the flow channels 1011a,1011b,1011c is received by the photodetector 1022a,1022b,1022c disposed on the opposite side of the respective flow channel.

On the other hand, the other measurement unit 1050 of FIG. 11 does not measure the optical properties of the chromogenic substance bound to the target material. In other words, the measurement unit 1050 has flow channels 1061a,1061b,1061c including a measurement optical path, which are used for the main measurement, the buffer measurement, and the reference measurement, respectively, and solutions for the first treatment and the second treatment are disposed before and after the flow channel. In other words, on the upstream side of the flow channels 1061a,1061b,1061c, container 1081a,1081b,1081c containing a solution including the first treatment solution, a buffer container 1082b containing the buffer solution and a reference reference solution 1082c including the reference solution are disposed corresponding to the respective flow channels. On the downstream side of the flow channels 1061a,1061b,1061c, the container 1091a,1091b,1091c containing a second treatment solution are disposed corresponding to the respective flow channels. Two light sources 1071a,1071b are arranged, and the light emitted from each passes through the flow channels 1061a,1061b,1061c, is received by the photodetectors 1072a,1072b,1072c disposed on the opposite side of the respective flow channels.

The original liquid introduced from the inlet 1012 is divided into a flow channel system of the main measurement flow channel 1011a of the measurement unit 1010 and a flow channel system of the main measurement flow channel 1061a of the measurement unit 1050. The pressure in the flow channel which is generated in this case can be released from the air vent 1013.

In the measurement unit 1050, containers 1081a,1081b,1081c containing a solution including the first treatment solution are disposed upstream of the flow channels 1061a,1061b,1061c, respectively, and containers 1091a,1091b,1091c containing a solution including the first treatment solution are disposed downstream of the flow channels 1061a,1061b,1061c, respectively. Two light sources 1071a,1071b are disposed, and the light emitted from each passes through the flow channels 1061a,1061b,1061c, is received by the photodetectors 1072a,1072b,1072c disposed on the opposite side of the respective flow channels.

The operation of the measurement units 1010, 1050 shown in FIG. 11 may be performed in the same or a similar manner to the measurement apparatus of FIG. 9.

The measurement apparatus of FIG. 11 may be, for example, a GA level measurement apparatus. The measurement unit 1010 can be used as an apparatus for measuring the concentration of albumin. The measurement unit 1050 can be used as an apparatus for measuring the concentration of glycoalbumin. The GA level can be obtained by dividing the glycoalbumin concentration by the albumin concentration.

An oxidizing agent may be contained as a pretreatment solution in the containers 1031a,1031b,1031c of pretreatment liquid of the measurement unit 1010. The containers 1041a,1041b, 1041c for the chromogenic substance of the measurement unit 1010 may contain BCP as the chromogenic substance. Then, a predetermined buffer solution may be contained in the buffer solution container 1031b, and a solution including albumin of a known concentration may be contained in the reference solution solution 1031c. For example, in the above configuration, the measurement unit 1010 can be used to derive the concentration of albumin as the measurement target substance in the original liquid from the optical measurement.

A ketone amine oxidase and a peroxidase (HRP) may be contained in the containers 1081a,1081b,1081c for the first treatment solution of the measurement unit 1050. A protease may be contained in the container 1091a,1091b,1091c for the second treatment solution. Then, a predetermined buffer solution may be contained in the container 1031b of the buffer solution, and a solution including glycoalbumin of a known concentration may be contained in the solution 1031c of the reference solution.

As an example, the concentration of albumin can be determined as follows. The buffer solution of the buffer container 1032b of FIG. 11 is mixed with the BCP of the chromogenic substance container 1041b. Thereafter, the absorbance measured in the buffer solution flow channel 1011b is output as the current or the converted voltage of the zero-reference absorbance output (photodiode 1022b). As a reference solution for the reference solution container 1032c, as an example, albumin 80 mg/dL of 20 % GA or the like may be used to measure the absorbance of a standard albumin concentration of tears. In the flow channel 1011a for the main measurement, a solution (e.g., tear fluid) including albumin of an unknown concentration to be measured is reacted with BCP to measure the absorbance. From these measurements, the concentration of albumin as a measurement target in the solution introduced into the apparatus can be determined.

On the other hand, the concentration of glycoalbumin (GA) can be determined from the absorbance measured in the measurement flow channels 1061a,1061b,1061c in the same manner as the concentration of albumin described above. For example, in a reference solution, fructosyl lysine included in 80 mg/dL of albumin of 20 % GA is known (for example, it is known to be 5 *µ*M). Thus, the concentration of GA can eventually be determined.

The ketoamine oxidase may be a dehydrogenase, may be a kinase, and may be an oxidase. The ketoamine oxidase may be fructosyl amino acid oxidase (FAOD), fructosyl peptide oxidase, fructosyl valylhistidine oxidase, fructosylamine oxidase, amadriase, fructosylamine deglyase or modified forms thereof.

In some embodiments, the ketoamine oxidase may be an oxidase which acts on an amino acid or peptide in which the *ε* -amino group is glycated. The amino acid may be lysine. By using an oxidase which selectively acts on an amino acid or peptide in which an *ε* -amino group is glycated, a glycoalbumin sensor can be constructed.

In some embodiments, the ketoamine oxidase may be an oxidase which acts on an amino acid or peptide in which the α-amino group is glycated. The amino acid may be valine. By using an oxidase that acts on an amino acid or peptide in which an *α* -amino group is glycated, a glycohemoglobin sensor or a glycohemoglobin A1c (HbA1c) sensor can be constructed.

"Protease" is a generic term for peptide bond hydrolyzing enzymes that hydrolyze and catabolize proteins and polypeptides. The protease may be an enzyme that degrades a protein into peptide fragments. When a protein includes a glycated amino acid residue, among the peptide fragments generated in the action of the protease, there may be peptide fragments including a glycated amino acid residue and peptide fragments not glycated at all.

"Protease" may be a protease derived from an animal, may be a protease derived from a plant, and may be a protease derived from a microorganism. The protease may be an exopeptidase and may be an endopeptidase. The protease may be an aspartic protease, a metal protease, a serine protease, or a thiol protease.

"Protease" may include a plurality of types or kinds of proteases and may include one type or kind of protease. For example, a protease may include one of a proteinase and a peptidase, and may include both. Mixing multiple proteases may increase the efficiency of degradation.

The animal-derived protease may be trypsin, chymotrypsin, elastase, bovine pancreatic protease, cathepsin, calpain, protease type-I, protease type-XX, aminopeptidase-N, carboxypeptidase, pancreatin (a mixture of multiple enzymes such as proteases and amylases), and the like.

The plant-derived protease may be papain, bromelain, gingipain, kallikrein, ficin, chymopapain, and the like.

The microbiota-derived protease may include Bacillus-derived proteases, Aspergillus-derived proteases, Penicillium-derived proteases, Streptomyces-derived proteases, Lysobacter-derived proteases, Yeast-derived proteases, Tritilatium-derived proteases, Thermus-derived proteases, Pseudomonas-derived proteases, Acromobacter-derived proteases, and the like.

The measurement apparatus shown in FIG. 11 can be used as a GA level measurement apparatus based on optical measurement. According to the present disclosure, a GA level measurement apparatus having a footprint of several centimeters by several centimeters or less can be manufactured on the basis of micro flow channel. The footprint may be smaller than or equal to a value such as 25 cm², 20 cm², 16 cm², 15 cm², 12 cm², 10 cm², 9 cm², 8 cm², 6 cm², and 4 cm².

In some embodiments, the measurement apparatus may be a protein measurement device or apparatus. In some embodiments, the measurement apparatus may be an albumin measurement device or apparatus.

The present disclosure includes a GA level measurement system. A GA level measurement system according to an embodiment may include an albumin measurement device and a glycated albumin measurement device. The albumin measurement device may include an absorbance measurement instrument of the present disclosure and may be configured to be connected to an absorbance measurement instrument. In some embodiments, the GA level measurement system may measure the albumin concentration in a solution using an albumin measurement device, measure the glycated albumin concentration using a glycated albumin measurement device, and calculate the GA level from the obtained albumin concentration and glycated albumin concentration. In some embodiments, the GA level measurement system may have an arithmetic processing unit that calculates a GA level from the obtained albumin concentration and glycated albumin concentration.

In some embodiments, the GA level measurement system may be configured to determine an albumin concentration and a glycated albumin concentration of tears.

The present disclosure includes a blood glucose level management system or a health management system. In some embodiments, a blood glucose management system or the like may include a GA measurement system. In some embodiments, the review control system may include a testing system, such as a CGM (Continuous Glucose Measurement (Continuous Glucose Monitoring)) apparatus or system.

In some embodiments, the blood glucose level management system may be configured to be connected to a GA level measurement system.

### 2. Measurement method

In some embodiments of the present disclosure, a method of measuring the concentration of a substance in a solution using an optical method such as an absorbance spectroscopy is provided. A measurement method using the measurement instrument of the present disclosure will be described in a nonlimiting or exemplary manner.

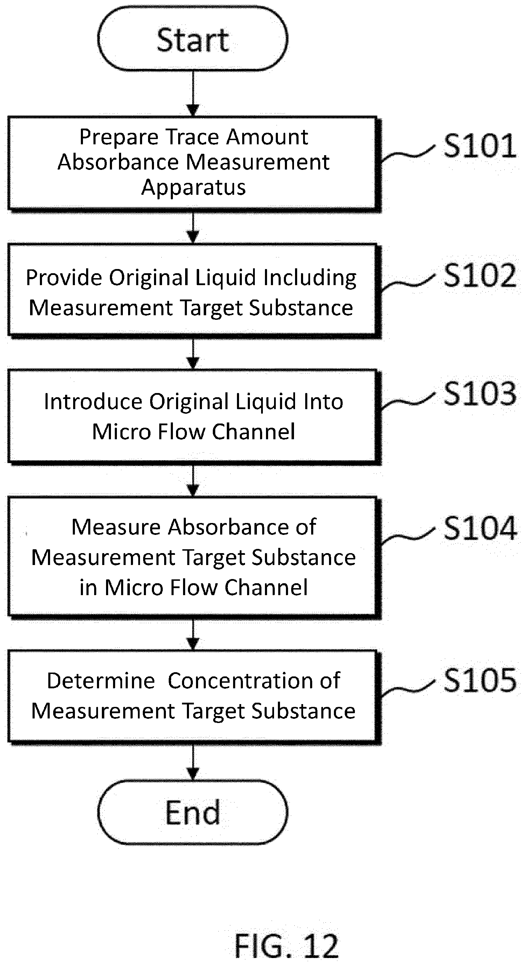

FIG. 12 shows a flow chart illustrating the steps of the measurement method according to one embodiment according to the present disclosure. First, an apparatus (which may be referred to as an absorbance measurement apparatus) for measuring the concentration of a substance in a solution by an absorbance spectroscopy according to the present disclosure is provided, prepared or the like (S101). In some embodiments, in the absorbance measurement apparatus the flow channel forms a micro flow channel at least in a portion of the optical path.

An original liquid including the measurement target substance is provided or prepared (S102). In some embodiments, a measurement target substance may be provided. The solution including the measurement target substance may be used for detection or measurement in a state at the time of acquisition, and processing such as dilution or replacement of the solution may be performed before introduction into the apparatus.

In some embodiments, the measurement target substance may be a biological substance such as a biomolecule, may be a chemical substance, and may be a mixture thereof. The measurement target substance may be a protein molecule present in a living body. The measurement target substance may be albumin. The original liquid including the measurement target substance may be tears or saliva or other body fluid. The measurement target substance may be albumin contained in tears or saliva.

Providing an original liquid including a measurement target substance may include obtaining tears or saliva of a human or other animal.

In FIG. 12, the preparation of the measurement target substance and the preparation of the solution (original solution) including the measurement target substance (S102) are described next to the preparation of the apparatus (S101), but in some embodiments, this order may be reversed, in some embodiments, the same timing may be adopted, and in some embodiments, the order may not be determined at all.

Next, the original solution including the measurement target substance is introduced into the micro flow channel of an absorbance measurement apparatus (S103). In some embodiments, the acquired solution may not be processed as it is, but may be introduced into an absorbance measurement apparatus. In some embodiments, after acquisition, the solution may be diluted, mixed with other solutions, physical filters, temperature treatment, chemical treatment, electrochemical treatment, electromagnetic wave treatment, or other treatments. The treatment for the solution may be performed before introduction into the absorbance measurement apparatus, may be performed after introduction into the absorbance measurement apparatus, may be performed before measurement, may be performed during measurement, and may be performed between a plurality of measurements. In some embodiments, the same, same types or different types of treatments may be performed at multiple timings.

Using an absorbance measurement apparatus, the absorbance of the measurement target substance introduced into the micro flow channel is measured or measured (S104).

The concentration of the measurement target substance in the micro flow channel can be determined from the absorbance (S105). If the concentration in the micro flow channel and the concentration before the introduction into the micro flow channel are substantially the same or substantially the same, the concentration determined directly by the measurement is the concentration of the measurement target substance in the original solution to be determined. If there is a relationship between the concentrations, the concentration of the measurement target substance in the original solution may be determined from the measured concentration based on the relationship. The relationship may be confirmed in advance by another measurement before the measurement.

In some embodiments, the absorbance of the measurement target substance in the micro flow channel may be determined, and the concentration of the measurement target substance in the original solution may be determined from the determined absorbance by conversion or another method. In some embodiments, the concentration of the measurement target substance in the original solution may be determined based on the output of the photodetector without calculating the absorbance of the measurement target substance in the micro flow channel as a value.

In some embodiments, a chromogenic substance may be used. In some embodiments, a chromogenic substance may be bound to the measurement target substance to be measured for concentration, and the absorbance of the chromogenic substance bound to the measurement target substance may be measured.

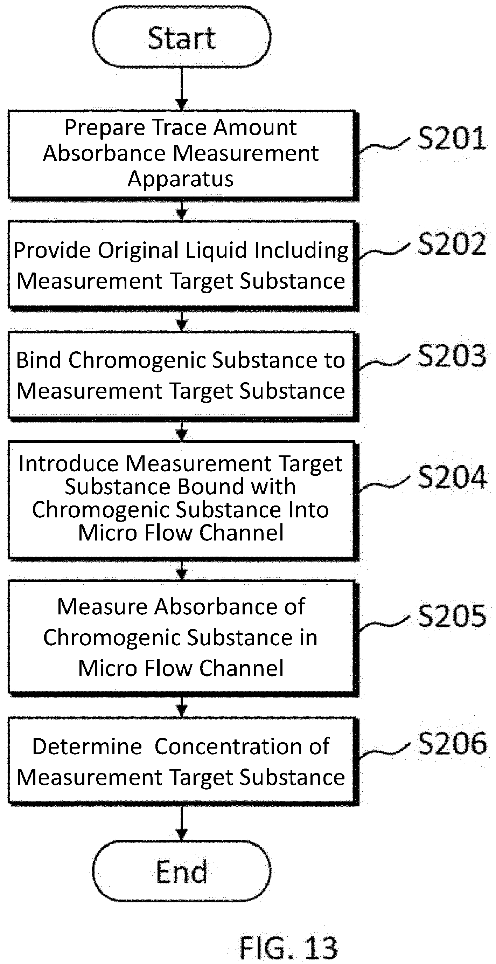

FIG. 13 shows a flow chart showing the steps of the measurement method according to the present disclosure according to one embodiment. An apparatus for measuring the concentration of a substance in a solution by an absorbance spectroscopy (which may be referred to as an absorbance measurement apparatus) according to the present disclosure is provided, prepared, or the like (S201).

An original liquid including the measurement target substance is provided or prepared (S202). In Fig. 13, the preparation of the measurement target substance and the preparation of the solution (original solution) including a measurement target substance (S202) are described next to the preparation of apparatus (S101), but in some embodiments, this order may be reversed, in some embodiments, the same timing may be adopted, and in some embodiments, the order may not be determined at all.

A chromogenic substance is bound to the measurement target substance (S203). In some embodiments, a solution including a bundle target substance and a solution including a chromogenic substance may be mixed.

The measurement solution is introduced into the micro flow channel of the apparatus (S204). Binding the chromogenic substance to the measurement target substance may be performed before introducing the measurement solution into the apparatus, and may be performed in the apparatus after introducing the measurement solution into the apparatus, and may be performed at other timings.

The absorbance of the chromogenic substance in the micro flow channel is measured using the absorbance spectroscopy (S205).

The concentration of the measurement target substance in the measurement solution (S206) is determined from the measured absorbance of the chromogenic substance. In some embodiments, the absorbance of the chromogenic substance in the micro flow may be determined, and the concentration of the measurement target substance in the original solution may be determined from the determined absorbance by conversion or another method. In some embodiments, the concentration of the measurement target substance in the original solution may be determined based on the output of the photodetector without calculating the absorbance of the chromogenic substance in the micro flow as a value. In some embodiments, the concentration of the measurement target substance in the corresponding original solution may be determined by conversion from the determined concentration during the measurement. In some embodiments, the measurement method may be an analysis of a protein by a dye-binding method, and may be a concentration measurement of a protein by a dye-binding method.

In some embodiments, the measurement target substance is or includes albumin, and the concentration of albumin in the original solution may be determined by measurement. In some embodiments, the chromogenic substance is or may include bromocresol purple. Bromocresol purple or a solution thereof and an original solution including albumin may be mixed. By this mixing, a solution including the albumin to which bromocresol purple is bound can be prepared as a measurement solution. In some embodiments, the absorbance of the bromocresol purple in the micro flow channel may be measured using an absorbance spectroscopy. In some embodiments, the concentration of the albumin in the measurement solution may be determined from the measured absorbance of the bromocresol purple.

In some embodiments, the absorbance may be measured at multiple wavelengths. In some embodiments, a wavelength characterizing the chromogenic substance (main wavelengths) and a wavelength in the wavelength region where the effect of absorption by the chromogenic substance is small or in the wavelength region of the background (sub-wavelengths) may be used. In some embodiments, bromocresol purple may be used as the chromogenic substance and measured at a main wavelength of 600 nm and a sub wavelength of 660 nm.

In some embodiments, the original solution and the oxidizing agent may be mixed prior to mixing the bromocresol purple or a solution thereof with the original solution. The oxidizing agent may be any agent that oxidizes albumin,
disulfide: 5,5'-dithiobis(2-nitrobenzoic acid)(DTNB), 2,2'-dithiobis(5-nitropyridine)(NPDS), 2,2'-dithiodipyridine(2-PDS), 4,4'-dithiodipyridine(4-PDS), 4,4'-dithiobis(1-azidobenzene)(DTBPA), oxidized glutathione, and the like, oxidizing agent: iodine, ferricyanide, iodosobenzoic acid,
iodate, salts of chloroacetate, mercury, zinc, and the like, alkylating agents: iodoacetic acid, chloroacetic acid, iodoacetamide, phenacyl chloride, and the like, Maleimide and its derivatives: maleimide, N-methylmaleimide, N-ethylmaleimide, N,N'-p-phoenix maleimide, and the like, thiophthalimide, and the like, among these, a disulfide such as DTNB, 2-PDS, 4-PDS, a maleimide, a maleimide derivative such as N-ethylmaleimide, or the like are preferably used. Further, these may be used alone or in combination as appropriate. Albumin exists in oxidized type and reduced type, and the specificity of BCP is altered depending on the type, which can lead to large errors. Therefore, by oxidizing all of the albumin using an oxidizing agent in advance, the accuracy of measurement can be increased.

In some embodiments, the concentration of albumin in the original solution may be determined from the concentration of albumin obtained in the measurement solution.

In some embodiments, the concentration of glycated albumin may be further determined. In some embodiments, a GA level may be determined based on the calculated albumin concentration and the calculated glycated albumin concentration. In some embodiments, a value obtained by dividing the calculated glycated albumin concentration by the calculated albumin concentration may be defined as a GA level. The calculation of the glycated albumin concentration is not limited in the present disclosure as there are various methods.

The present disclosure includes methods of disease state management, including glycemic management, methods of health management of preliminary or healthy persons with disease states, methods of disease prevention, and other methods of health care management. In some embodiments, these healthcare management methods may comprise determining albumin concentration using an optical measurement apparatus having a micro flow channel. In some embodiments, the healthcare management method may be a method of providing healthcare information, a non-invasive diabetes risk management method, a diabetic complication prevention method, or the like.

The present disclosure also includes the following embodiments. A01 An apparatus for measuring a substance in a solution, comprising:
a light source;
a micro flow channel including at least a portion of an optical path of light emitted from the light source and extending along the optical path; and a photodetector for detecting light passed through the micro flow channel.

### A01b

An apparatus for measuring a concentration of a substance in a solution by absorption spectrophotometry, comprising:
a flow channel being configured to contain a solution, defining an optical path and
having a volume of 20 *µ*L or smaller;
a light source; and
a photodetector for detecting an electromagnetic wave emitted from the light source and having passed through the optical path defined in the flow channel.

### A02

The apparatus according to embodiment A01,
wherein the micro flow channel has a volume of 20 *µ*L or smaller.

### A03

The apparatus according to embodiment A02,
wherein the micro flow channel has a volume of 10 *µ*L or smaller.

### A04

The apparatus according to embodiment A03,
wherein the micro flow channel has a volume of 5 *µ*L or smaller.

### A05

The apparatus according to any one of embodiments A01 to A04,
wherein a cross-sectional dimension of the optical path of the micro flow channel is 3 mm or smaller.

### A06

The apparatus according to embodiment A05,
wherein the cross-sectional dimension of the optical path of the micro flow channel is 2 mm or smaller.

### A07

The apparatus according to embodiment A06,
wherein the cross-sectional dimension of the optical path of the micro flow channel is 1 mm or smaller.

### A08

The apparatus according to any one of embodiments A01 to A07, further comprising:
a second photodetector configured to measure light emitted by the light source outside the light path (an adjustment photodetector); and
a second photodetector controller for receiving information related to the luminance of the light measured by the second photodetector and controlling the light emission amount of the light source so as to be substantially constant in time.

### A09

The apparatus according to any one of embodiments A01 to A08, comprising a photodetector controller configured to receive information related to the luminance measured by the photodetector(first photodetector, measurement photodetector), and to make the light emitting amount of the light source to be substantially constant in time.

### A10

The apparatus according to any one of embodiments A01 to A09, further comprising a temperature controller for regulating the temperature of the solution in the micro flow channel.

### A11

The apparatus according to any one of embodiments A01 to A09, further comprising a heater disposed proximate the micro flow channel and configured to control the temperature of the solution.

### A12

The apparatus according to any one of embodiments A01 to A11, wherein at least a portion of the member forming the micro flow channel has a higher heat capacity than the solution in the micro flow channel.

### A13

The apparatus according to any one of embodiments A01 to A12, further comprising a shield configured to substantially prevent light from other than the light source from being detected by the photodetector.

### A14

The apparatus according to any one of embodiments A01 to A13, wherein the member forming the micro flow channel includes a shielding material.

### A15

The apparatus according to any one of embodiments A01 to A14, wherein the inner wall of the micro flow channel is hydrophilic.

### A16

The apparatus according to any one of embodiments A01 to A15, wherein the micro flow channel has an inlet and an outlet for the solution.

### A17

The apparatus according to embodiment A16, wherein the inlet and the outlet are arranged so that air bubbles are not substantially formed in the micro flow channel when the solution is introduced.

### A18

The apparatus according to embodiment A16, wherein the inlet and the outlet are disposed at a position of the entrance end and the exit end of the optical path in the micro flow channel.

### A19

The apparatus according to embodiment A16, wherein the inlet and the outlet are arranged such that no dead space is substantially formed in the micro flow channel.

### A31

The apparatus according to embodiments A01 to A19, wherein the light source has a plurality of peak wavelengths.

### A32

The apparatus according to embodiment A31, wherein the light source includes a plurality of light sources each having a different peak wavelength.

### A33

The apparatus according to embodiment A32, further comprising a light source control mechanism for introducing light from only one light source of the plurality of light sources into the light path.

### A34

The apparatus according to embodiment A32, further comprising a drive control apparatus for controlling an emission of the plurality of light sources.

### A35

The apparatus according to embodiment A33, wherein the light source control mechanism includes a drive control apparatus for controlling the emission of the plurality of light sources.

### A51

The apparatus according to any one of embodiments A01 to A35, further comprising a reference measurement flow channel including at least a portion of the reference measurement optical path and extending along the reference measurement optical path.

### A52

The apparatus according to embodiment A51, further comprising a reference measurement optical system configured to propagate light emitted from the light emitter along the reference measurement optical path in the reference measurement flow channel and receive light by the photodetector.

### A53

The apparatus according to embodiment A51, comprising:
a reference measurement light source for propagating light along the reference measurement optical path in the reference measurement flow channel; and
a reference measurement photodetector for detecting light emitted from the light source and propagating in the reference measurement flow channel along the reference measurement optical path.

### A54

The apparatus according to any one of embodiments A51 to A53, wherein a reference solution is contained in the reference measurement flow channel.

### A55

The apparatus according to any one of embodiments A01 to A54, wherein the apparatus is an absorbance optical apparatus by absorbance spectroscopy.

### A61

The apparatus according to embodiment A01,
wherein the optical path includes:
a first optical path in which light emitted from the light source propagates, and
a second optical path in which light emitted from the light source propagates,
wherein the micro flow channel includes a first micro flow channel extending along the first optical path and a second micro flow channel extending along the second optical path, and
wherein the photodetector includes a first photodetector for detecting light passed through the first micro flow channel and a second photodetector for detecting light passed through the second micro flow channel.

### A62

The apparatus according to embodiment A01,
wherein the light source includes a first light source and a second light source, wherein the optical path includes a first optical path in which light emitted from the first light source propagates, and a second optical path in which light emitted from the second light source propagates,
wherein the micro flow channel includes a first micro flow channel extending along the first optical path and a second micro flow channel extending along the second optical path, and
wherein the photodetector includes a first photodetector for detecting light passed through the first micro flow channel and a second photodetector for detecting light passed through the second micro flow channel.

### A63

An apparatus for measuring a substance in a solution, comprising:
a first light source;
a second light source;
a first micro flow channel including at least a portion of a first optical path of light emitted from the first light source and extending along the first optical path;
a second micro flow channel including at least a portion of a second optical path of light emitted from the second light source and extending along the second optical path;
a first photodetector for detecting light passed through the first micro flow channel; and
a second photodetector for detecting light passed through the second micro flow channel.

### A64

The apparatus according to any one of embodiments A61 to A63, further comprising:
a first container being configured to be fluidly coupled to the first flow channel,
having an inlet for introducing a measurement target substance, and containing a solution to be mixed with the measurement target substance; and
a second container being configured to be fluidly coupled to the second flow channel, and containing a solution to be mixed with the measurement target substance.

### A65

The apparatus according to embodiment A64, further comprising a mixing mechanism for mixing the measurement target substance and the solution to be mixed with the measurement target substance.

### A101

The apparatus according to any one of embodiments A01 to A65, further comprising:
a chromogenic substance container containing a chromogenic substance;
an inlet for the measurement target substance;
a mechanism for mixing the measurement target substance and the chromogenic substance introduced from the inlet; and
a mechanism for feeding the mixed solution of the measurement target substance and the chromogenic substance to the micro flow channel.

### A102

The apparatus according to embodiment A101,
wherein the measurement target substance includes albumin, and wherein the chromogenic substance includes bromocresol purple.

### A103

The apparatus according to embodiment A102,
wherein the measurement target substance is albumin in a tear fluid.

### A104

The apparatus according to embodiment A102 or A103, further comprising an oxidant container containing an oxidizing agent.

### A105

The apparatus according to embodiment A104, further comprising a mechanism for mixing the tear fluid and the oxidizing agent.

### A201

A GA level measurement system comprising:
an albumin measurement device according to any one of embodiments A101 to A105;
a glycated albumin measurement device; and
a processing unit capable of calculating a GA level based on an albumin concentration obtained using the albumin measurement device and a glycated albumin concentration obtained using the glycated albumin measurement device.

### A202

The GA level measurement system according to embodiment A201, configured to determine the albumin concentration of tear fluid and the glycated albumin concentration. A203
A blood glucose level management system comprising the GA level measurement system according to embodiment A201 or A202. A204
A blood glucose level management system configured to be connected to the GA level measurement system according to embodiment A201 or A202. B01 A method for measuring a concentration of a substance in a solution, comprising providing the apparatus according to any one of embodiments A01 to A204; providing a solution including a measurement target substance;
introducing the solution including the measurement target substance into the micro flow channel of the apparatus;
measuring the absorbance of the substance in the micro flow channel using absorbance spectroscopy; and
determining the concentration of the measurement target substance in the solution from the measured absorbance associated with the substance.

### B01b

A method for measuring a concentration of a substance in a solution, comprising: providing the apparatus according to any one of embodiments A01 to A204; providing a solution including a measurement target substance;
introducing the solution including the measurement target substance into the micro flow channel of the apparatus;
performing an optical measurement on the solution in the micro flow channel; and determining from the measurement, the concentration of the measurement target substance in the solution.

### B02

A method for measuring the concentration of a substance in a solution, comprising: providing the apparatus according to any one of embodiments A01 to A204; providing an original solution including a measurement target substance;
binding a chromogenic substance to the measurement target substance; providing a measurement solution including the measurement target substance to which the chromogenic substance is bonded;
introducing the measurement solution into the micro flow channel of the apparatus;
measuring the absorbance of the chromogenic substance in the micro flow channel using absorbance spectroscopy; and
determining from the measured absorbance of the chromogenic substance the concentration of the measurement target substance in the measurement solution.

### B02b

A method for measuring a concentration of a substance in a solution, comprising providing the apparatus according to any one of embodiments A01 to A204; providing an original solution including a measurement target substance;
binding a chromogenic substance to the measurement target substance; providing a measurement solution including the measurement target substance to which the chromogenic substance is bonded;
introducing the measurement solution into the micro flow channel of the apparatus;
performing an optical measurement on the solution in the micro flow channel;
determining from the measurement the concentration of the measurement target substance in the solution; and
determining from the measurement the concentration of the measurement target substance in the measurement solution.

### B03

The method according to embodiment B02b, further comprising:
determining the concentration of the measurement target substance in the corresponding original solution by conversion from the concentration determined in the measurement.

### B04

A method for measuring a concentration of albumin, comprising:
providing the apparatus according to any one of embodiments A01 to A204;
providing an original solution including albumin;
mixing bromocresol purple with the original solution including the albumin, to bind it to the albumin;
preparing a measurement solution including the albumin to which the bromocresol purple is bound;
introducing the measurement solution into the micro flow channel of the apparatus;
performing an optical measurement on the solution in the micro flow channel; measuring from the measurement the concentration of the bromocresol purple in the micro flow channel; and
determining from the measured concentration of bromocresol purple the concentration of the albumin in the measurement solution.

### B05

The method according to embodiment B04, wherein the original solution including the albumin is tear fluid or saliva.

### B06

The method according to embodiment B04 or B05, further comprising mixing the original solution and an oxidizing agent, prior to said mixing the bromocresol purple and the original liquid.

### B07

The method according to any one of embodiments B04 to B06, further comprising determining from the concentration of the albumin in the measurement solution the concentration of the albumin in the original solution.

### B11

A GA level measurement method comprising:
calculating a concentration of albumin using the method according to any one of embodiments B04 to B06;
calculating a concentration of glycated albumin;
determining a GA level based on the calculated albumin concentration and the calculated glycated albumin concentration.

### B12

A blood glucose level management method comprising determining the GA level using the GA level measurement method according to embodiment B11.

While several embodiments and examples of the present disclosure have been described above, these embodiments and examples illustrate the present disclosure. For example, each of the embodiments described above has been described in detail in order to explain the present disclosure in an easy-to-understand manner, and dimensions, configurations, materials, and circuits may be additionally changed as necessary. Embodiments in which one or more features of the present disclosure described above are arbitrarily combined are also included in the scope of the present disclosure. It is intended that the appended claims cover numerous modifications to the embodiments without departing from the spirit and scope of the present disclosure. Accordingly, the embodiments and examples disclosed herein have been shown by way of illustration and should not be considered as limiting the scope of the present disclosure.

### Reference Signs List

100 Optical measurement apparatus
110 Main body
111 Flow channel
112 Inlet
113 Outlet
121 Light source
122 Photodetector
123 Optical path
124 Light entrance window
125 Light exit window
131 Measurement target substance
132 Solution
200 Optical absorbance measurement apparatus
210 Housing
211 Flow channel
221 a, 221b Light source
222 Photodetector
223 Optical path
224 Light entrance window
226 Half mirror
231 Solution
232 Measurement target substance
300 Optical measurement apparatus
310 Main body
311 Flow channel
312 Inlet
313 Outlet
321 Light source
322 Photodetector
323 Optical path
324 Light window
325 Light exit window
326 Adjustment photodetector
327 Controller
329 Photodetector output circuit
331 Measurement target substance
332 Solution
341 Heater
342 Temperature sensor
343 Temperature controller
350 Processing circuit (CPU)
361 Housing (flow channel housing)
362 Fixing portion
363 Fitting
400 Optical measurement apparatus
410 Main body
411a,411b Flow channels
421 Light source
422a,422b Photodetectors
423a,423b Optical paths
424 Light entrance window
425 Light exit window
426 Adjustment photodetector
427 Beam splitter
428 Mirror
431 Measurement target substance
432 Original liquid
433 Chromogenic substance
434 Measurement solution
435 Reference measurement solution (Measurement solution)
460 Flow channel member
461 Inlet
462 Mixing vessels
463 Mixing mechanism
464 Reservoir
465 Air hole
466 Sealing lid
600 Measurement apparatus
601 Inlet for the target measurement target substance
602 Chromogenic substance solution container
603 Mixing portion
604 Measurement light path
700 Measurement apparatus
701 Inlet for the target measurement target substance
702 Chromogenic substance solution container
703 Second mixing portion
704 First measurement optical path
705 Pretreatment solution container
706 First mixing portion
707 Second measurement optical path
800 Measurement apparatus
801 Introduction port for the target measurement target substance
802 Chromogenic substance solution container
803 Second mixing portion
804 Measurement light path
805 Pretreatment solution container
806 First mixing portion
900 Measurement apparatus
911a, 911b, 911c Flow channels
912 Inlet
913 Air vent
921a, 921b Light source
922a,922b,922c Photodetector
931a, 931b, 931c Container
932b Buffer container
932c Reference solution container
941a, 941b, 941c Containers
1000 Measurement apparatus
1010,1050 Measurement unit
1011a,1011b,1011c Flow channel
1012 Inlet
1013 Air vent
1021a, 1021b Light source
1022a,1022b,1022c Photodetectors
1031a, 1031b, and 1031c Containers
1032b buffer container
1032c Reference solution container
1041a, 1041b, 1041c Containers
1061 a, 1061b, 1061c Flow channels
1071a, 1071b Light source
1072a,1072b,1072c Photodetectors
1081a, 1081b,1081c Containers
1091a, 1091b, 1091c Containers

## Claims

1. An apparatus for measuring a substance in a solution, comprising:
a light source;
a micro flow channel including at least a portion of an optical path of light emitted from the light source and extending along the optical path; and
a photodetector for detecting light passed through the micro flow channel.

2. The apparatus according to Claim 1,
wherein the micro flow channel has a volume of 20 *µ*L or smaller.

3. The apparatus according to Claim 2,
wherein the micro flow channel has a volume of 10 *µ*L or smaller.

4. The apparatus according to Claim 3,
wherein the micro flow channel has a volume of 5 *µ*L or smaller.

5. The apparatus according to any one of Claims 1 to 4,
wherein a cross-sectional dimension of the optical path of the micro flow channel is 3 mm or smaller.

6. The apparatus according to Claim 5,
wherein the cross-sectional dimension of the optical path of the micro flow channel is 2 mm or smaller.

7. The apparatus according to Claim 6,
wherein the cross-sectional dimension of the optical path of the micro flow channel is 1 mm or smaller.

8. The apparatus according to any one of Claims 1 to 7, further comprising:
a second photodetector configured to measure light emitted by the light source outside the light path (an adjustment photodetector); and
a second photodetector controller for receiving information related to the luminance of the light measured by the second photodetector and controlling the light emission amount of the light source so as to be substantially constant in time.

9. The apparatus according to any one of Claims 1 to 8, comprising a photodetector controller configured to receive information related to the luminance measured by the photodetector(first photodetector, measurement photodetector), and to make the light emitting amount of the light source to be substantially constant in time.

10. The apparatus according to any one of Claims 1 to 9 further comprising a temperature controller for regulating the temperature of the solution in the micro flow channel.

11. The apparatus according to any one of Claims 1 to 9, further comprising a heater disposed proximate the micro flow channel and configured to control the temperature of the solution.

12. The apparatus according to any one of Claims 1 to 11, wherein at least a portion of the member forming the micro flow channel has a higher heat capacity than the solution in the micro flow channel.

13. The apparatus according to any one of Claims 1 to 12, further comprising a shield configured to substantially prevent light from other than the light source from being detected by the photodetector.

14. The apparatus according to any one of Claims 1 to 13, wherein the member forming the micro flow channel includes a shielding material.

15. The apparatus according to any one of Claims 1 to 14, wherein the inner wall of the micro flow channel is hydrophilic.

16. The apparatus according to any one of Claims 1 to 15, wherein the micro flow channel has an inlet and an outlet for the solution.

17. The apparatus according to Claim 16, wherein the inlet and the outlet are arranged so that air bubbles are not substantially formed in the micro flow channel when the solution is introduced.

18. The apparatus according to Claim 16, wherein the inlet and the outlet are disposed at a position of the entrance end and the exit end of the optical path in the micro flow channel.

19. The apparatus according to Claim 16, wherein the inlet and the outlet are arranged such that no dead space is substantially formed in the micro flow channel.

20. The apparatus according to Claims 1 to 19, wherein the light source has a plurality of peak wavelengths.

21. The apparatus according to Claim 20, wherein the light source includes a plurality of light sources each having a different peak wavelength.

22. The apparatus according to Claim 21, further comprising a light source control mechanism for introducing light from only one light source of the plurality of light sources into the light path.

23. The apparatus according to Claim 21, further comprising a drive control apparatus for controlling an emission of the plurality of light sources.

24. The apparatus according to Claim 22, wherein the light source control mechanism includes a drive control apparatus for controlling the emission of the plurality of light sources.

25. The apparatus according to any one of Claims 1 to 24, further comprising a reference measurement flow channel including at least a portion of the reference measurement optical path and extending along the reference measurement optical path.

26. The apparatus according to Claim 25, further comprising a reference measurement optical system configured to propagate light emitted from the light emitter along the reference measurement optical path in the reference measurement flow channel and receive light by the photodetector.

27. The apparatus according to Claim 25, comprising:
a reference measurement light source for propagating light along the reference measurement optical path in the reference measurement flow channel; and
a reference measurement photodetector for detecting light emitted from the light source and propagating in the reference measurement flow channel along the reference measurement optical path.

28. The apparatus according to any one of Claims 25 to 27, wherein a reference solution is contained in the reference measurement flow channel.

29. The apparatus according to any one of Claims 1 to 28, wherein the apparatus is an absorbance optical apparatus by absorbance spectroscopy.

30. The apparatus according to Claim 1,
wherein the optical path includes:
a first optical path in which light emitted from the light source propagates, and
a second optical path in which light emitted from the light source propagates,
wherein the micro flow channel includes a first micro flow channel extending along the first optical path and a second micro flow channel extending along the second optical path, and
wherein the photodetector includes a first photodetector for detecting light passed through the first micro flow channel and a second photodetector for detecting light passed through the second micro flow channel.

31. The apparatus according to Claim 1
wherein the light source includes a first light source and a second light source, wherein the optical path includes a first optical path in which light emitted from the first light source propagates, and a second optical path in which light emitted from the second light source propagates,
wherein the micro flow channel includes a first micro flow channel extending along the first optical path and a second micro flow channel extending along the second optical path, and
wherein the photodetector includes a first photodetector for detecting light passed through the first micro flow channel and a second photodetector for detecting light passed through the second micro flow channel.

32. An apparatus for measuring a substance in a solution, comprising:
a first light source;
a second light source;
a first micro flow channel including at least a portion of a first optical path of light emitted from the first light source and extending along the first optical path;
a second micro flow channel including at least a portion of a second optical path of light emitted from the second light source and extending along the second optical path;
a first photodetector for detecting light passed through the first micro flow channel; and
a second photodetector for detecting light passed through the second micro flow channel.

33. The apparatus of any one of Claims 30 to 32, further comprising:
a first container being configured to be fluidly coupled to the first flow channel, having an inlet for introducing a measurement target substance, and containing a solution to be mixed with the measurement target substance; and
a second container being configured to be fluidly coupled to the second flow channel, and containing a solution to be mixed with the measurement target substance.

34. The apparatus according to Claim 31, further comprising a mixing mechanism for mixing the measurement target substance and the solution to be mixed with the measurement target substance.

35. The apparatus according to any one of Claims 1 to 34, further comprising:
a chromogenic substance container containing a chromogenic substance;
an inlet for the measurement target substance;
a mechanism for mixing the measurement target substance and the chromogenic substance introduced from the inlet; and
a mechanism for feeding the mixed solution of the measurement target substance and the chromogenic substance to the micro flow channel.

36. The apparatus according to Claim 35,
wherein the measurement target substance includes albumin, and
wherein the chromogenic substance includes bromocresol purple.

37. The apparatus according to Claim 36,
wherein the measurement target substance is albumin in a tear fluid.

38. The apparatus according to Claim 36 or 37, further comprising an oxidant container containing an oxidizing agent.

39. The apparatus according to Claim 38, further comprising a mechanism for mixing the tear fluid and the oxidizing agent.

40. A GA level measurement system comprising:
an albumin measurement device according to any one of Claims 35 to 39;
a glycated albumin measurement device; and
a processing unit capable of calculating a GA level based on an albumin concentration obtained using the albumin measurement device and a glycated albumin concentration obtained using the glycated albumin measurement device.

41. The GA level measurement system according to Claim 40, configured to determine the albumin concentration of tear fluid and the glycated albumin concentration.

42. A blood glucose level management system comprising the GA level measurement system according to Claim 40 or 41.

43. A blood glucose level management system configured to be connected to the GA level measurement system according to Claim 40 or 41.

44. A method for measuring a concentration of a substance in a solution, comprising
providing the apparatus according to any one of Claims 1 to 43;
providing a solution including a measurement target substance;
introducing the solution including the measurement target substance into the micro flow channel of the apparatus;
measuring the absorbance of the substance in the micro flow channel using absorbance spectroscopy; and
determining the concentration of the measurement target substance in the solution from the measured absorbance associated with the substance.

45. A method for measuring a concentration of a substance in a solution, comprising
providing the apparatus according to any one of to Claims 1 to 43;
providing an original solution including a measurement target substance;
binding a chromogenic substance to the measurement target substance; providing a measurement solution including the measurement target substance to which the chromogenic substance is bonded;
introducing the measurement solution into the micro flow channel of the apparatus;
performing an optical measurement on the solution in the micro flow channel; determining from the measurement the concentration of the measurement target substance in the solution; and
determining from the measurement the concentration of the measurement target substance in the measurement solution.

46. The method according to Claim 45, further comprising:
determining the concentration of the measurement target substance in the corresponding original solution by conversion from the concentration determined in the measurement.

47. A method for measuring a concentration of albumin, comprising:
providing the apparatus according to any one of Claims 1 to 43;
providing an original solution including albumin;
mixing bromocresol purple with the original solution including the albumin, to bind it to the albumin;
preparing a measurement solution including the albumin to which the bromocresol purple is bound;
introducing the measurement solution into the micro flow channel of the apparatus;
performing an optical measurement on the solution in the micro flow channel;
measuring from the measurement the concentration of the bromocresol purple in the micro flow channel; and
determining from the measured concentration of bromocresol purple the concentration of the albumin in the measurement solution.

48. The method according to Claim 47, wherein the original solution including the albumin is tear fluid or saliva.

49. The method according to Claim 48 or 49, further comprising mixing the original solution and an oxidizing agent, prior to said mixing the bromocresol purple and the original liquid.

50. The method according to any one of Claims 47 to 49, further comprising determining from the concentration of the albumin in the measurement solution the concentration of the albumin in the original solution.

51. A GA level measurement method comprising:
calculating a concentration of albumin using the method of any one of Claims 47 to 50;
calculating a concentration of glycated albumin; and
determining a GA level based on the calculated albumin concentration and the calculated glycated albumin concentration.

52. A blood glucose level management method comprising determining the GA level using the GA level measurement method according to Claim 51.
